# NEUE EUROPÄISCHE PATENTSCHRIFT

(11) **EP 0 684 236 B2**
(45) Veröffentlichungstag und Bekanntmachung des Hinweises auf die Entscheidung über den Einspruch: **26.12.2007**
(45) Hinweis auf die Patenterteilung: 24.06.1998
(21) Anmeldenummer: 95106220.7
(22) Anmeldetag: 25.04.1995
(51) Int. Cl.: C07D 239/48

(54) **Verfahren zur Herstellung von N-(2-Amino-4,6-dichlorpyrimidin-5-yl)formamid**
Process of preparation of N-(2-Amino-4,6-dichloropyrimidin-5-yl)formamide
Procédé de préparation du N-(2-Amino-4,6-dichloropyrimidin-5-yl)formamide

(30) Priorität: 27.04.1994 CH 129994
(43) Veröffentlichungstag der Anmeldung: 29.11.1995
(60) Teilanmeldung: 97114001.7
(73) Patentinhaber: Lonza AG, CH-3945 Gampel/Wallis (CH)
(72) Erfinder: Stucky, Gerhard, Dr., CH-3902 Brig-Glis (Kanton Wallis) (CH); Imwinkelried, René, Dr., CH-3904 Naters (Kanton Wallis) (CH)
(74) Vertreter: Weinhold, Peter

(56) Entgegenhaltungen:
- EP-A- 0 552 758
- WO-A-91/01310
- SYNTHESIS, Bd. 7, 1990 Seiten 587-589, M. LEGRAVEREND ET AL. 'A New Route to 2,5-Diamino-4,6-dichloropyrimidine, A Key Precursor of 9-Substituted Guanines'
- J. ORG. CHEM., Bd. 40, Nr. 21, 1975 Seiten 3141-3142, C. TEMPLE, JR. ET AL. 'Preparation of 2,5-Diamino-4,6-dichlorpyrimidine'
- CHEMICAL ABSTRACTS, vol. 89, no. 25, 18.Dezember 1978 Columbus, Ohio, US; abstract no. 215347s, C. TEMPLE, JR. ET AL. 'Preparation of 2,5-diamino-4,6-dichloropyrimidine via N-(4,6-dichloro-5-nitropyrimidin-2-yl)acet amide: The preparation of 2-aminopyrimidine intermediates' Seite 591; & NUCLEIC ACID CHEM., Nr. 1, 1978 Seiten 47-52,

## Beschreibung

Die Erfindung betrifft ein Verfahren zur Herstellung von N-(2-Amino-4,6-dichlorpyrimidin-5-yl)formamid. Diese Verbindung ist ein wertvolles Zwischenprodukt zur Herstellung von antiviralen Nukleotidderivaten (PCT-Anmeldung WO 91/01 310).

Bekannt sind bisher N-5-geschützte 2,5-Diamino-4,6-dichlorpyrimidine, die als Zwischenprodukte zur Herstellung von antiviralen Nukleotiddervaten dienen (EP-A 0 552 758). Diese Verbindungen haben jedoch den Nachteil, dass sie sich schlecht zu den entsprechenden Nukleotidderivaten umsetzen lassen.

Aufgabe der Erfindung war es daher, ein wirtschaftliches Verfahren zur Herstellung von N-(2-Amino-4,6-dichlorpyrimidin-5-yl)formamid bereitzustellen, mit welchem die entsprechenden Nukleotidderivate in guter Ausbeute erhalten werden.

Diese Aufgabe wurde mit dem neuen Verfahren zur Herstellung von N -(2-Amino-4,6-dichlorpyrimidin-5-yl)formamid der Formel 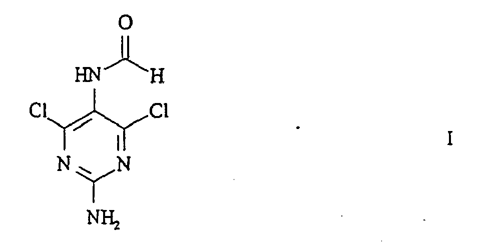 nach Anspruch 1 gelöst.

In der ersten Verfahrensstufe wird ein Aminomalonester der allgemeinen-Formel 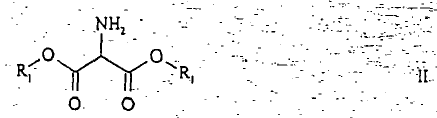 worin R₁ eine C₁-C₆-Alkylgruppe bedeutet, oder dessen Salz, mit Guanidin oder dessen Salz in Gegenwart einer Base zum 2,5-Diamino-4,6-dihydroxypyrimidin der Formel 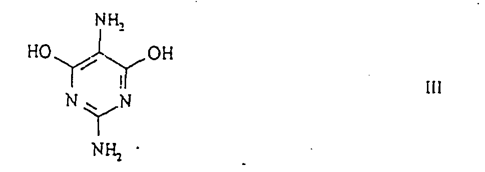 oder dessen Salz, cyclisiert.
Die als Edukte eingesetzten Aminomalonester der allgemeinen Formel II können auf bekannte Weise durch Amidierung der entsprechenden Malonester-Derivate erhalten werden.

Als Base wird zweckmässig, entsprechend zur EP-A 0 552 758, ein Alkalimetallalkoholat wie beispielsweise Natrium- oder Kaliummethanolat oder Natrium- oder Kaliumethanolat verwendet. Vorzugsweise wird in situ gebildetes Natriummethanolat in Methanol oder Natriumethanolat in Ethanol angewendet.

Als Salze des Aminomalonesters und des Guanidins werden zweckmässig deren Hydrochlorid-bzw. Hydrobromid-Salze verwendet.

Zweckmässig erfolgt die Cyclisierung bei einer Temperatur zwischen Raumtemperatur und Rückflusstemperatur des entsprechenden Lösungsmittels, Vorzugsweise bei Rückflusstemperatur.

Nach einer üblichen Umsetzungszeit zwischen 2 und 6 h kann dar gegebenenfalls das Zwischenprodukt gemäss Formel III durch übliche Aufbereitungsmethoden isoliert werden Vorzugsweise erfolgt die Synthese des Endproduktes gemäss Formel I ohne Isolation des Zwischenproduktes gemäss Formel III.

Die zweite Verfahrensstufe erfolgt derart, dass man das Zwischenprodukt gemäss Formel III oder dessen Salz mit einem Chlorierungsmittel in Gegenwart eines Amids der allgemeinen Formel 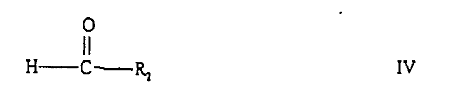 zu einem 4,6-Dichlorpyrimidin der allgemeinen Formel 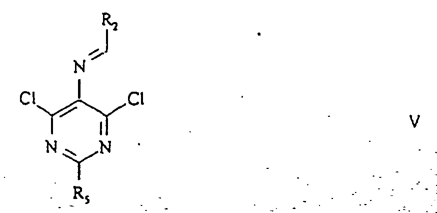 chloriert.

Der Substituent R₅ bedeutet -NH₂. Der Substituent R₂ bedeutet entweder
- einen 5- oder 6-gliedrigen N-Heterocycloalkylrest, der gegebenenfalls am Stickstoffatom substituiert ist, wie beispielsweise Piperidinyl-, Morpholinyl-. Thiornorpholinyl-, Pyrrolidinyl-,N-Methylpiperazinyl, vorzugsweise Piperidinyl- oder Pyrrolidinyl, oder
- NR₃R₄, worin R₃ und R₄ gleich oder verschieden sind und eine C₁-C₆-Alkylgruppe wie beispielsweise Methyl-, Ethyl-, Propyl-, Isopropyl-, Butyl-, Pentyl- oder Heuyl-, vorzugsweise Methyl-, oder eine Benzylgruppe bedeuten.

Der N-Heterocycloalkylrest kann zusätzlich andere Heteroatome, wie z.B. ein Sauerstoffatom, erthalten.

Demzufolge können als Amide der Formel IV N,N-Dimethylformamid, N,N-Diethylformamid, N,N-Diisopropyfformamid, N-Formylpiperidin, N-Formylmorpholin N-Formylthiomorpholin, N,N-Methyl-formylpiperazin oder N,N-Dibenzylformamid, vorzugsweise N,N-Dimethylformamid, N-Formylpiperidin oder N,N-Dibenzylformamid angewendet werden.

Als Salze des Zwischenproduktes gemäss Formel III werden zweckmässig dessen Hydrochlorid- bzw. Hydrobromid-Salze oder dessen Alkalimetallsalze wie beispielsweise dessen Natrium- oder Kaliumsalz verwendet.

Als Chlorierungsmittel können fachmännisch übliche angewendet werden wie beispielsweise Phosphoroxychlorid, Thionylchlorid, Sulfurylchlorid, Phosphortrichlorid, Phosphorpentachlorid, Phosgen oder Diphosgen. Vorzugsweise wird als Chlorierungsmittel Phosphoroxychlorid verwendet.

Zweckmäßig werden das Chlorierungsmittel und das Amid (IV) im Molverhältnis 1 zu 0,55 bis 1 zu 10, vorzugsweise im Molverhältnis 1 zu 0,55 bis 1 zu 1, angewendet.

Die Chlorierung wird zweckmässig bei einer Temperatur von 50°C bis Rückflusstemperatur des entsprechenden Lösungsmittels durchgeführt.

Als Lösungsmittel für die Chlorierung kann das zuvor beschriebene Amid angewendet werden Die Chlorierung kann jedoch auch noch zusätzlich in einem inerten Lösungsmittel durchgeführt werden. Als inerte Lösungsmittel können beispielsweise Toluol, Xylol, Chloroform, Dichlormethan, Dichlorethan oder Chlorbenzol angewendet werden, vorzugsweise Toluol oder Dichlorethan.

Nach einer üblichen Umsetzungszeit von 3 bis 24 h kann das entsprechende 4,6-Dichlorpyrimidin der allgemeinen Formel V (R₅ = -NH₂) auf fachmännische Weise isoliert werden. Bevorzugte Vertreter der 4,6-Dichlorpyrimidine (V, R₅ = -NH₂) sind 4,6-Dichlor-N'-(dimethylaminomethylen)pyrimidin-2,5-diamin und 4,6-Dichlor-N'-(piperidin-1-ylmethylen)pyrimidin-2,5-diamin.

- Je nach Wahl der Reaktions- oder Aufarbeitungsbedingungen können auch Vorstufen dieser 4,6-Dichlorpyrimidine (V) isoliert werden. Diese Vorstufen sind ebenfalls durch die allgemeine Formel V definiert. R₅ bedeutet dann -NH-CH=O oder -N=CH-R₂, worin R₂ die genannte Bedeutung hat. Bevorzugte Vertreter solcher 4,6-- Dichlorpyrimidine sind 4,6-Dichlor-N,N'-bis(dimethylaminomethyten)pyrimidin-2,5-diamin, 4,6-Dichlor-N,N'-bis(piperidin-1-ylmethylen)pyrimidin-2,5-diamin bzw. N-[4,6-Dichlor-5-(dimethylaminomethylenamino)pyrimidin-2-yl]-formamid.

4,6-Dichlorpyrimidine der Formel V gemäß Anspruch 9 sind in der Literatur nicht beschrieben und daher als neue Zwischenprodukte zur Herstellung von N-(2-Amino-4,6-dichlorpyrimidin-5-yl)formamid ebenfalls Bestandteil der Erfindung.

In der dritten Verfahrensstufe werden die 4,6-Dichlorpyrimidine der allgemeinen Formel V mit einer wässrigen Carbonsaure der allgemeinen Formel

R₆-COOH VI

worin R₅ eine C₁-C₆-Alkylgmppe, verzweigt oder unverzweigt oder eine C₃-C₆-Cyctoalkylgruppe bedeutet, zum Endprodukt gemäss Formel umgesetzt.

Als Carbonsäure kann Essigsäure. Propionsäure, Buttersäure, Pentancarbonsäure, Hexancarbonsäure, lsobuttersäure. Pivalinsäure. Cyclopropancarbonsäure, Cyclopentancarbonsäure oder Cyclohexancarbonsäure angewendet werden. Zweckmässig wird Essigsäure, Propionsäure oder Pivalinsäure angewendet.

Zweckmässig wird die Carbonsäure in einer Konzentration von 20 bis 70 Vol.%, vorzugsweise von 25 bis 50 Vol.%, angewendet

Zweckmässig wird die Umsetzung in der dritten Stufe bei einer Temperatur von 50 bis 100°C, vorzugsweise bei einer Temperatur von 70 bis 90°C, durchgeführt

Nach einer üblichen Umsetzungszeit von 1 bis 10 h kann dann das Endprodukt gemäss Formel I durch fachmännisch übliche Aufarbeitungsmethoden isoliert werden. Das neue Endprodukt gemäss Formel 1 kann im Unterschied zu den bekannten N-S-geschützten-2,5-Diamino-4,6-dichlorpyrimidinen (EP-A 0 552 758) auf einfache Weise und in guter Ausbeute in das entsprechende Nukleotidderivat überfürt werden.

### Beispiele

### Beispiel 1 Herstellung von N-(2-Amino-4,6-dichlorpyrimidin-5-yl)formamid

### 1.1 Herstellung von 4 6-Dichlor-N'-(dimethylaminomethylen)pyrimidin-2,5 diamin

Eintopfverfahren:
Eine Suspension von 25 g(117 mmol) Aminomalonester-hydrochlorid in 50 ml Methanol wurde auf 10°C abgekühlt und mit 21,07 g Natriummethanolat (30%ig in Methanol) versetzt Diese Suspension wurde zu einem Gemisch von 63.2 g (351 mmol) Natriummethanolat (30%ige Lösung in Methanol) und 12,55 g (128,7 mmol) Guanidin-hydrochlorid in 50 ml Methanol zugetropft. Das Reaktionsgemisch wurde zum Rückfluss erhitzt und anschliessend für 16 Stunden bei dieser Temperatur gerührt. Zur warmen Suspension wurden anschliessend 13,5 g (370 mmol) HCl-Gas eingeleitet. Anschliessend wurde das Methanol abdestilliert. Während der Destillation wurden langsam insgesamt 200 ml Toluol zugetropft. Nachdem alles Methanol herausdestilliert war, wurden 71,6 g (468 mmol) POCl₃ und anschliessend bei 80°C 34,2 g (468 mmol) Dimethylformamid zugetropft. Man liess 17,5 Stunden bei 80°C rühren, kühlte dann auf Raumtemperatur ab und versetzte langsam mit 64,7 g K₂CO₃, gelöst in 150 ml Wasser. Man erwärmte erneut für 5 Stunden auf 50°C. Danach wurde mit 30%iger NaOH-Lösung der pH auf 7 gestellt, das Reaktionsgemisch gekühlt und das Produkt filtriert. Nach Waschen mit Wasser und Trocknen am Vakuum erhielt man 23,2 g (85%) reines Produkt als hellbraunen Feststoff.

| | |
|---|---|
| ¹H-NMR (DMSO, 400 MHz) δ: | 2,9-3,0 (2 s, 6H); |
| | 6.9 (s, 2H): |
| | 7,6 (s, 1 H). |
| ¹³C-NMR (DMSO, 100 MHz): | 33,5; |
| | 39,3; |
| | 130,3; |
| | 153,5; |
| | 157,0; |
| | 157,1. |
| m.p.: | 195°C (dec.). |

### 1.2 Herstellung von N-(2-Amino-4,6-dichlorpyrimidin-5-yl)formamid

a) Eine Lösung von 2,35 g (10 mmol) des Produktes aus 1.1 in 15 g 50%iger wässriger Propionsäure wurde für 7 Stunden bei 70°C gerührt Anschliessend wurde abgekühlt und das Produkt filtriert. Nach Waschen mit Wasser und Trocknen am Vakuum erhielt man 1,66 g eines weissen Feststoffs. Dieser wurde in 50 ml 2M K₂CO₃-Lösung aufgeschlämmt und 2 Stunden bei Raumtemperatür gerührt Anschliessend wurde filtriert, mit Wasser gewaschen und das Produkt am Vakuum getrocknet. Man erhielt 1,33 g (64%) reines Produkt als fast weissen Feststoff.

| | |
|---|---|
| ¹H-NMR (DMSO, 300 MHz) δ: | 9,6-10.1 (b, 1H); |
| | 8,3 und 8,0 (2 s, 1 H); |
| | 7,7 und 7,6 (2 s, 2H). |

b) Analog zu a) wurde anstatt Propionsäure Pivalinsäure als Carbonsaure angewendet und das Produkt entsprechend aufgearbeitet. Die Ausbeute betrug 70%.

### Beispiel 2 Herstellung von 4,6-Dichlor-N'(dimethylaminomethylen)pyrimidin-2,5-diamin

### 2.1 Herstellung von 4,6-Dichlor-N,N'-bis(dimethylaminemethylen)pyrimidin-2,5-diamin

Eine Suspension von 4,46 g (25 mmol) Diaminodihydroxypyrimidin-hydrochlorid in 45 ml Toluol und 15,33 g (100 mmol) Phosphoroxychlorid wurde auf 90°C erwärmt. Innerhalb von 45 Minuten wurden 7,31 g (100 mmol) Dimethylformamid zugetropft Anschliessend wurde 20 Stunden bei 90°C gerührt Man liess abkühlen und versetzte das Reaktionsgemisch langsam mit 100 g 10% iger K₂CO₃-Lösung. Anschliessend wurden 19,5 g festes K₂CO₃ zugegeben, damit der pH auf 7 anstieg. Das Produkt wurde mit drei Portionen Essigester extrahiert. Die gemeinsamen organischen Phasen wurden über MgSO₄ getrocknet und am Rotationsverdampfer eingeengt Man erhielt 6,44 g eines hellbraunen Feststoffs. Ausbeute: 89%.

| | |
|---|---|
| ¹H-NMR (DMSO, 400 MHz) δ: | 2.9-3.1 (4s, 12 H); |
| | 7,6 (s, 1H); |
| | 8.5 (s, 1H). |
| | |
| ¹³C-NMR (DMSO, 100 MHz): | 33,5; |
| | 39,4; |
| | 40,4; |
| | 134,2; |
| | 153,0; |
| | 156.7; |
| | 158,0; |
| | 159.1. |
| | |
| m.p.: | 121,5- 123°C. |
| | |
| CHN: | ber. für C₁₀H₁₄Cl₂N₆: C 41,54, H 4,88. N 29,06; gef: C 41,4, H 4,58. N 28,6. |

### 2.2 Herstellung von N-[4,6-Dichlor-5-(dimethylaminomethylenamino)pyrimidin-2-yl]-formamid

Eine Suspension von 3 g (10 mmol) des Produktes aus 2.1 in 10 ml 50%iger wässriger Essigsäure wurde 4,5 Stunden bei Raumtemperatur gerührt Anschliessend wurde das Produkt filtriert und 2 x mit je 10 ml Wasser gewaschen. Nach Trocknung am Vakuum erhielt man 2.18 g (83%) reines Produkt als weissen Feststoff.

| | |
|---|---|
| ¹H-NMR (DMSO, 400 MHz) δ: | 2,9-3,1 (2 s, 6H); |
| | 7,7 (s, 1H): |
| | 9,2 (d, 1H); |
| | 11,2 (d, 1H). |
| ¹³ C.NMR (DMSO. 100 MHz): | 33,6; |
| | 39,6; |
| | 136,9; |
| | 149,6; |
| | 153,4; |
| | 156,9; |
| | 162,5. |
| | |
| m.p.: | 172,5 - 174°C. |
| | |
| CHN: | ber. für C₈H₉Cl₂N₅O: C 36,66, H3,46, N 26,72; gef: C 36,7, H 3,07, N 25,9. |

### 2.3 Herstellung von 4,6-Dichlor-N'-(dimethylaminomethylen)pyrimidin-2,5-diamin

Eine Lösung von 1,85 g (7,1 mmol) des Produktes aus 2.2 in 25 ml 10%iger Salzsäure wurde auf 40°C erwärmt und bei dieser Temperatur 1,5 Stunden gerührt. Das Reaktionsgemisch wurde abgekühlt und mit 2M K₂CO₃ der pH auf 8,7 gestellt. Das ausgefalleng Produkt wurde filtriert und mit Wasser gewaschen. Nach Trocknung am Vakuum erhielt man 1,52 g (91%) reines Produkt als weissen Feststoff.

Spektroskopische Daten analog oben.

### 2.4 Herstellung von 4,6-Dichlor-N'-(dimethylaminomethylen)pyrimidin-2,5-diamin

Eintopfverfähren:
Eine Suspension von 4,46 g (25 mmol) Diaminodihydroxypyrimidin-hydrochlorid in 90 ml Toluol und 15,33 g (100 mmol) Phosphoroxychlorid wurde auf 80°C erwärmt. Innerhalb von 60 Minuten wurden 7,31 g (100 mmol) Dimethylformamid zugetropft Anschliessend wurde 16 Stunden bei 80°C gerührt Man liess abkühlen und versetzte das Reaktionsgemisch mit 100 ml Wasser. Mit insgesamt 8.4 g Na₂CO₃ wurde der pH auf 10 gestellt. Man erwärmte auf 40°C und rührte das Reaktionsgemisch für 4 Stunden bei dieser Temperatur. Anschliessend wurde auf Raumtemperatur abgekühlt, mit 30%iger NaOH-Lösung neutralisiert und das Produkt filtriert Nach Waschen mit Wasser und Trocknen am Vakuum erhielt man 5,5 g (95%iges) Produkt als beigen Feststoff Dies entspricht einer Ausbeute von 89%.
Spektroskopische Daten analog oben.

### Beispiel 3 Herstellung-von 4,6-Dichlor-N'-(piperidin-1-ylmethylen)pyrimidin-2,5-diamin

### 3.1 Herstellung von 4,6-Dichlor-N,N'-bis(piperidin-yimethylen)pyrimidin-2,5-diamin

Eine Suspension von 3,57 g (20 mmol) Diaminodihydroxypyrimidin-hydrochlorid in 70-mlToluol und 12,27 g (80 mmol) Phosphoroxychlorid wurde auf 80°C erwärmt. Innerhalb von 60 Minuten wurden 9,05 g (80 mmol) 1-Formylpiperidin zugetropft Anschliessend wurde 22 Stunden bei 80°C gerührt. Man liess abkühlen und versetzte das Reaktionsgemisch mit 100 ml 1M K₂CO₃-Lösung. Anschliessend wurde mit NaOH der pH auf 7 eingestellt. Das Produkt wurde mit drei Portionen Essigester extrahiert. Die gemeinsamen organischen Phasen wurden über MgSO₄ getrocknet und am Rotationsverdampfer eingeengt Man erhielt 10,87 g eines Öls, welches noch sehr viel N-Formylpiperidin enthielt. Das Produkt konnte durch Aufschlämmen in Hexan und anschliessende Filtration gereinigt werden. Ausbeute: >90%.

| | |
|---|---|
| ¹H-NM (DMSO, 300 MHz) δ: | 8.5 (s, 1H); |
| | 7,7 (s, 1H); |
| | 3,4-3,8 (m, 8H); |
| | 1,5-1,9 (m, 12H). |

### 3.2 Herstellung von 4,6-Dichlor-N'-(piperidin-1-ylmethylen)pyrimidin-2,5-diamin

Eine Lösung von 9,9 g (18,2 mmol) des Produktes aus 3.1 in 73 g 10%iger HCl wurde zuerst 4,5 Stunden bei Raumtemperatur und anschliessend 2 Stunden bei 47°C gerührt. Man kühlte ab und stellte den pH mit 30%iger NaOH auf 7 ein. Das Produkt wurde filtriert, mit Wasser gewaschen und am Vakuum getrocknet. Man erhielt 4,68 g (88%) Produkt als hellbraunen Feststoff.

| | |
|---|---|
| ¹H-NMR (DMSO, 300 MHz) δ: | 7.55 (s, 1H); |
| | 7,4 (s, 2H); |
| | 3,2-3,7 (m, 4H); |
| | 1,5-1,8 (m, 6H). |

### Beispiel 4 Weiterumsetzung von N-(2-Amino-4,6-dichlorpyrimidin-5-yl)formamid zu 2-Amino-9-butyl-6-chlor-purin

### 4.1 Herstellung von N-(2-Amino-4-butylamin-6-chlorpyrimidin-5-yl)formamid

Eine Lösung von 0.43 g (2 mmol) N-(2-Amino-4,6-dichlorpyrimidin-5-yl)formamid und 0,31 g (4,2 mmol) n-Butylamin in 10 ml Tetrahydrofüran wurde 17 Stunden bei Raumtemperatur gerührt. Anschliessend wurde das Reaktionsgemisch mit Wasser versetzt, und das Produkt mit Essigester extrahiert Nach Trocknen der organischen Phase über MgSO₄ und Einengen am Rotationsverdampfer erhielt man 0,49 g eines weissen Feststoffs, der durch Umkristallisation in Toluol gereinigt werden konnte. Man erhielt 0,46 g reines Produkt, was einer quantitativen Ausbeute entspricht.

| | |
|---|---|
| ¹H-NMR (DMSO, 300 MHz) δ: | 9,0 und 8.6 (s und d, 1H); |
| | 8,1 und 7,8 (s und d, 1 H); |
| | 7,0 und 6.75 (2 t, 1H); |
| | 6,5 und 6.4 (2 s, 2H): |
| | 3,3-3,4 (m, 2H): |
| | 1,4-1.6 (m, 2H); |
| | 1,2-1,4 (m, 2M: |
| | 0,9 (t, 3H). |

### 4.2 Herstellung von 2-Amino-9-butyl-6-chlor-purin

Eine Suspension von 0,51 g (2 mmol) des Produktes aus 4.1 in 10 ml Diethoxymethylacetat wurde 3,5 Stunden zum Rückfluss erhitzt Anschliessend wurde vollständig eingeengt und der Rückstand mit 30 ml 0.5M HCl-Lösung versetzt. Nach 3 Stunden bei Raumtemperatur wurde die gelbe Lösung mit NaOH auf einen pH-Wert von 8 gestellt und die entstandene Suspension 3 x mit Essigester extrahiert Die gemeinsamen organischen Phasen wurden getrocknet und am Rotationsverdampfer eingeengt Man erhielt 0,46 g (97%) des gewünschten Produktes mit einem Gehalt von 95% (nach ¹H-NMR).

| | |
|---|---|
| ¹H-NMR (DMSO, 300 MHz) δ: | 8.2 (s, 1H); |
| | 6,9 (s, 2H): |
| | 4,05 (t, 2H); |
| | 1,6-1,9 (m,2H); |
| | 1,1-1,4 (m, 2H); |
| | 0.9 (t, 3H). |

### 4.3 Umsetzung von 5(N-Ethoxycarbonyl)-2-amino-4,6 dichloropyrimdin zu 2-Amino-9-buty-6-chlor-7,9-dihydrocuring-8-on (Vergleichsbeispiel)

Als Vergleichsbeispiel wurde 5-(N-Ethoxycarbonyl)-2-amino-4,6-dichlorpyrimidin als Derivat eines N-5-geschützten-2.5-Diamino-4,6-dichlorpyrimidin (EP-A 0 552 758) unter analogen Bedingungen wie in Beispiel 4 umgesetzt.

Unter diesen Bedingungen wurde jedoch nicht 2-Amino-9-butyl-6-chlor-purin sondern 2-Amino-9-butyl-6-chlor-7,9-dihydropurin-8-on erhalten.

## Patentansprüche

1. Verfahren zur Herstellung von N-(2-Amino-4,6-dichlorpyrimidin-5-yl) formamid der Formel 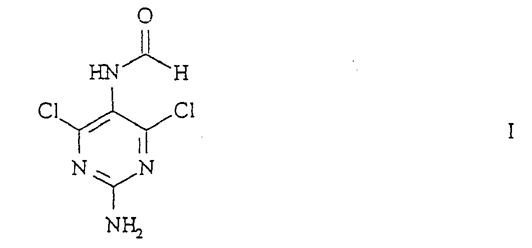 **dadurch gekennzeichnet, daß** man in der ersten Stufe einen Aminomalonester der allgemeinen Formel 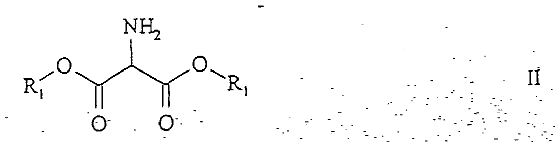 worin R₁ eine C₁-C₆-Alkylgruppe bedeutet, oder dessen Salz, mit Guanidin oder dessen Salz in Gegenwart einer Base zum 2,5-Diamino-4,6-dihydroxypyrimidin der Formel 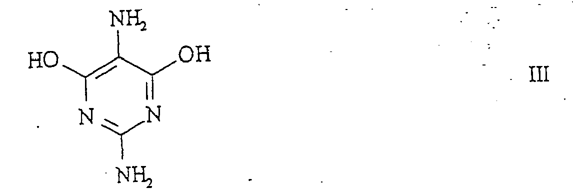 oder dessen Salz cyclisiert, dieses dann in der zweiten Stufe mit einem Chlorierungsmittel in Gegenwart eines Amids der allgemeinen Formel 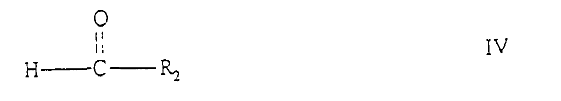 worin R₂ einen 5- oder 6-gliedrigen N-Heterocycloalkylrest, der gegebenenfalls am Stickstoffatom substituiert ist, oder -NR₃R₄, worin R₃ und R₄ gleich oder verschieden sind und eine C₁-C₆-Alkylgruppe oder eine Benzylgruppe darstellen, bedeutet, zu einem 4,6-Dichlorpyrimidin der allgemeinen Formel 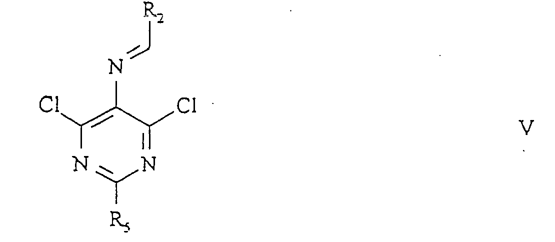 chloriert, worin R₂ die genannte Bedeutung hat und R₅ -NH₂ bedeutet, und dieses dann in der dritten Stufe mit einer wässrigen Carbonsäure der allgemeinen Formel
R₆-COOH VI
worin R₆ eine C₁-C₆-Alkylgruppe, verzweigt oder unverzweigt öder eine C₃-C₆-Cycloalkylgruppe bedeutet, zum Endprodukt gemäß - Formel I umsetzt.

2. Verfahren nach Patentanspruch 1, **dadurch gekennzeichnet, daß** man die Umsetzung ohne Isolation der Zwischenprodukte der Formel III durchführt.

3. Verfahren nach Patentanspruch 1 oder 2, **dadurch gekennzeichnet, daß** man in der ersten Stufe als Base ein Alkalimetallalkoholat verwendet.

4. Verfahren nach einem der Patentansprüche 1 bis 3, **dadurch gekennzeichnet, daß** man in der zweiten Stufe als Chlorierungsmittel Phosphoroxychlorid verwendet.

5. Verfahren nach einem der Patentansprüche 1 bis 4, **dadurch gekennzeichnet, daß** man in der zweiten Stufe als Amid Dimethylformamid, N-Formylpiperidin oder N,N-Dibenzylformamid verwendet.

6. Verfahren nach einem der Patentansprüche 1 bis 5, **dadurch gekennzeichnet, daß** man die Chlorierung in der zweiten Stufe bei einer Temperatur von 50°C bis Rückflußtemperatur durchführt.

7. Verfahren nach einem der Patentansprüche 1 bis 6, **dadurch gekennzeichnet, daß** man in der dritten Stufe als aliphatische Carbonsäure Essigsäure, Propionsäure oder Pivalinsäure verwendet.

8. Verfahren nach einem der Patentansprüche 1 bis 7, **dadurch gekennzeichnet, daß** man die Umsetzung in der dritten Stufe bei einer Temperatur von 50 bis 100°C durchführt.

9. 4,6-Dichlor-pyrimidine der allgemeinen Formel 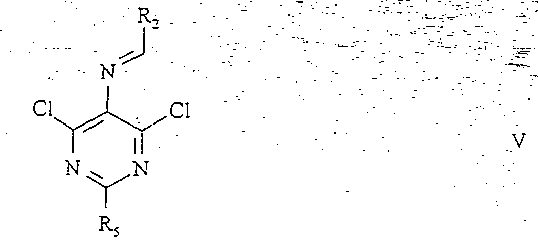 worin R₂ einen 5- oder 6-gliedrigen N-Heterocycloalkylrest, der gegebenenfalls am Stickstoffatom substituiert ist, oder -NR₃R₄, worin R₃ und R₄ gleich oder verschieden sind und eine C₁-C₆-Alkylgruppe oder eine Benzylgruppe darstellen, bedeutet, und R₅ -NH₂, -NH-CH = O oder -N = CH-R₂ ist, worin R₂ die genannte Bedeutung hat, mit der Einschränkung, daß R₂ nicht -NR₃R₄ ist, worin R₃ und R₄ jeweils C₁-C₆-Alkyl ist, wenn R₅ -NH₂ oder -N = CH-NR₃R₄ ist, worin R₃ und R₄ jeweils C₁-C₆-Alkyl ist.

10. 4,6-Dichlor-N'-(piperidin-1-ylmethylen)pyrimidin-2,5-diamin.

## Claims

1. Method for producing N-(2-amino-4,6-dichloropyrimidin-5-yl)formamide having the formula 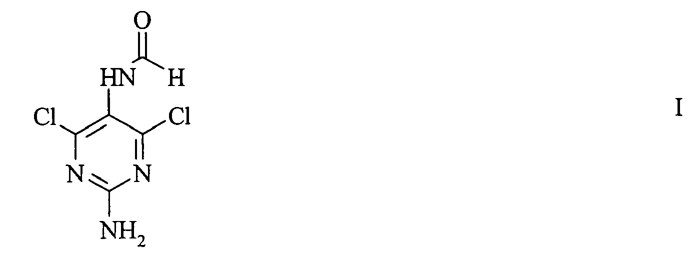 **characterised in that** in the first step an aminomalonic ester having the general formula 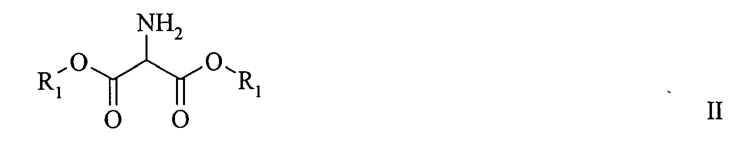 wherein R₁ designates a C₁-C₆ alkyl group, or its salt, is cyclised with guanidine, or its salt, in the presence of a base into 2,5-diamino-4,6-dihydroxypyrimidine having the formula 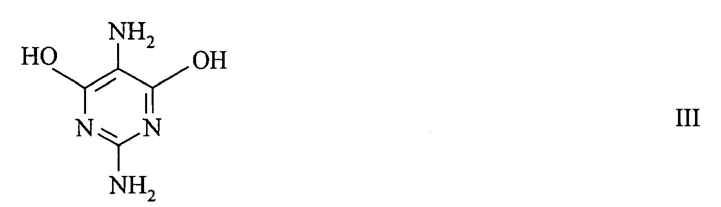 or its salt, which is then chlorinated in the second step, with a chlorinating agent in the presence of an amide having the general formula 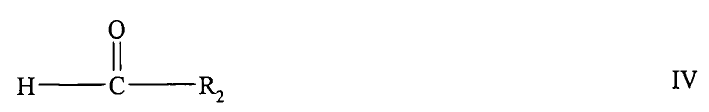 wherein R₂ is a 5-link or 6-link N-heterocycloalkyl radical optionally substituted at the nitrogen atom, or -NR₃R₄, wherein R₃ and R₄ are identical or different and represent a C₁-C₆ alkyl group or a benzyl group, into a 4,6-dichloropyrimidine having the general formula 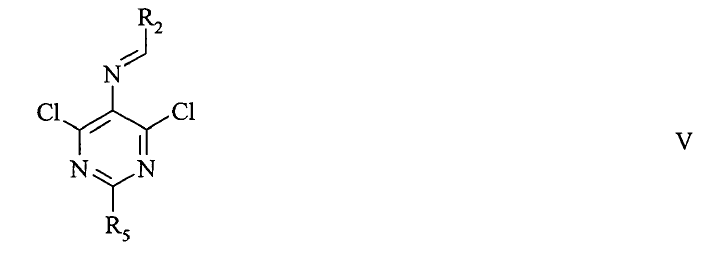 wherein R₂ has the named meaning and R₅ designates -NH₂, which is then in a third step reacted into the final product according to Formula I with an aqueous carboxylic acid having the general formula
R₆-COOH VI
wherein R₆ designates a branched or linear C₁-C₆ alkyl group, or a C₃-C₆ cycloalkyl group.

2. The method of claim 1, **characterised in that** the reaction is carried out without isolating the intermediate products of Formula III.

3. The method of claim 1 or 2, **characterised in that** in the first step an alkali metal alcoholate is used as a base.

4. The method of one of claims 1 to 3, **characterised in that** in the second step phosphorus oxychloride is used as a chlorinating agent.

5. The method of one of claims 1 to 4, **characterised in that** in the second step dimethylformamide, N-formylpiperidine or N,N-dibenzylformamide is used as an amide.

6. The method of one of claims 1 to 5, **characterised in that** chlorination in the second step is carried out at a temperature from 50°C to reflux temperature.

7. The method of one of claims 1 to 6, **characterised in that** in the third step acetic acid, propionic acid or pivalic acid is used as an aliphatic carboxylic acid.

8. The method of one of claims 1 to 7, **characterised in that** reaction in the third step is carried out at a temperature from 50°C to 100°C.

9. 4,6-Dichloro-pyrimidine having the general formula 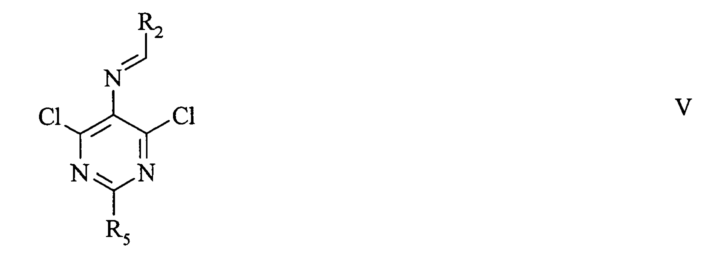 wherein R₂ designates a 5-link or 6-link N-heterocycloalkyl radical optionally substituted at the nitrogen atom, or -NR₃R₄, wherein R₃ and R₄ are identical or different and represent a C₁-C₆ alkyl group or a benzyl group, and R₅ is -NH₂, -NH-CH=O or -N=CH-R₂, wherein R₂ has the named meaning, with the restriction that R₂ is not -NR₃R₄, wherein R₃ and R₄ each are C₁-C₆ alkyl when R₅ is -NH₂ or -N=CH-NR₃R₄, wherein R₃ and R₄ each are C₁-C₆ alkyl.

10. 4,6-Dichloro-N'-(piperidin-1-ylmethylene)pyrimidine-2,5-diamine.

## Revendications

1. Procédé de fabrication de N-(2-amino-4,6-dichloropyrimidin-5-yl) formamide de formule 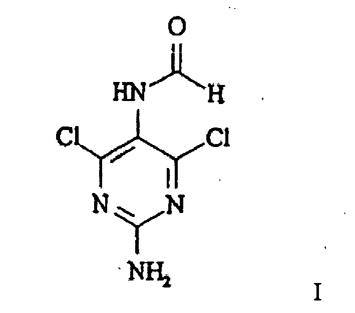 **caractérisé en ce que** dans la première étape, on cyclise un ester d'aminomalone de formule générale 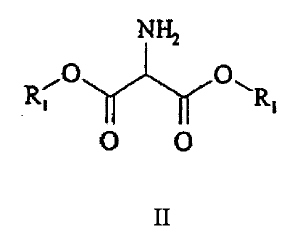 dans lequel R₁ représente un groupement alkyle C₁-C₆, ou son sel, avec de la guanidine ou son sel en présence d'une base, pour donner la 2,5-diamino-4,6-dihydroxypyrimidine de formule 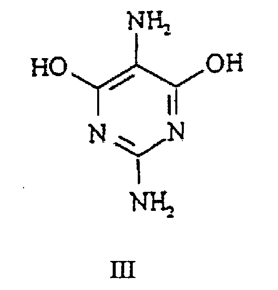 ou son sel ; ceci est ensuite chloré dans une deuxième étape avec un agent de chloration en présence d'un amide de formule générale 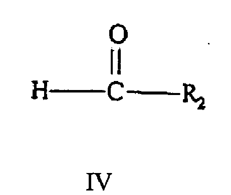 dans lequel R₂ représente un radical N-hétérocycloalkyle à 5 ou 6 membres, qui le cas échéant est substitué au niveau de l'atome d'azote, ou -NR₃R₄ où R₃ et R₄ sont identiques ou différents et représentent un groupement alkyle C₁-C₆ ou un groupement benzyle, pour donner une 4,6-dichloropyrimidine de formule générale 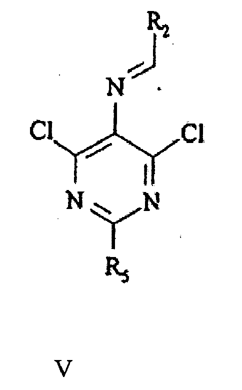 dans laquelle R₂ a la signification citée et R₅ représente -NH₂, et celle-ci est ensuite transformée dans une troisième étape par un acide carboxylique aqueux de formule générale
R₆-COOH VI
dans laquelle R₆ représente un groupement alkyle C₁-C₆, linéaire ou ramifié, ou un groupement cycloalkyle C₃C₆, pour donner le produit final selon la formule I.

2. Procédé selon la revendication 1 **caractérisé en ce qu'**on effectue la transformation sans isoler les produits intermédiaires de formule III.

3. Procédé selon la revendication 1 ou 2 **caractérisé en ce qu'**on utilise comme base à la première étape un alcoolate de métal alcalin.

4. Procédé selon une des revendications 1 à 3 **caractérisé en ce qu'**on utilise comme agent chlorant à la deuxième étape un oxychlorure de phosphore.

5. Procédé selon une des revendications 1 à 4 **caractérisé en ce qu'**on utilise comme amide à la deuxième étape le diméthylformamide, la N-formylpipéridine ou le N,N-dibenzylformamide.

6. Procédé selon une des revendication 1 à 5 **caractérisé en ce qu**'on effectue la chloration à la deuxième étape à une température de 50°C jusqu'à la température de reflux.

7. Procédé selon une des revendications 1 à 6 **caractérisé en ce qu**'on utilise comme acide carboxylique aliphatique à la troisième étape l'acide acétique, l'acide propionique ou l'acide pivalinique.

8. Procédé selon une des revendications 1 à 7 **caractérisé en ce qu**'on effectue la réaction à la troisième étape à une température de 50 jusqu'à 100°C.

9. 4,5-dichloropyrimidine de formule générale 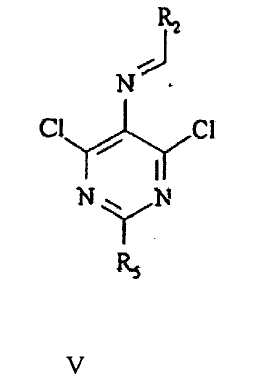 dans lequel R₂ représente un radical N-hétérocycloalkyle à 5 ou 6 membres, qui le cas échéant est substitué au niveau de l'atome d'azote, ou -NR₃R₄ où R₃ et R₄ sont identiques ou différents et représentent un groupement alkyle C₁-C₆ ou un groupement benzyle, et R₅ représente -NH₂, -NH-CH=O ou -N=CH-R₂ pour lequel R₂ a la signification citée sous réserve que R₂ ne soit pas -NR₃R₄ où R₃ et R₄ sont l'un ou l'autre un groupement alkyle C₁-C₆ si R₅ représente -NH₂ ou -N=CH-NR₃R₄ où R₃ et R₄ sont l'un ou l'autre un groupement alkyle C₁-C₆.

10. 4,6-dichloro-N' -(pipéridin-1-yl-méthylèn)-pyrimidin-2,5-diamine.
